(19) Europäisches Patentamt
European Patent Office
Office européen des brevets

(11) Publication number: **0 196 904 B1**

# (12) EUROPEAN PATENT SPECIFICATION

(45) Date of publication of patent specification: **17.06.92**

(51) Int. Cl.[5]: **C08L 71/02**, A61K 7/48, A61K 31/77

(21) Application number: **86302368.5**

(22) Date of filing: **27.03.86**

## (54) Perfluoropolyether compositions.

(30) Priority: **29.03.85 IT 2016185**

(43) Date of publication of application:
**08.10.86 Bulletin 86/41**

(45) Publication of the grant of the patent:
**17.06.92 Bulletin 92/25**

(84) Designated Contracting States:
**AT BE CH DE FR GB LI NL SE**

(56) References cited:
**WO-A-83/02620**

**CHEMICAL PATENTS INDEX BASIC ABSTRACTS JOURNAL, Section A, Plasdoc., abstract no. 85-084003, Derwent Publications Ltd, London; & JP-A-60 034 730 (NIPPON MECTRON K.K.) 22-02-1985**

**CHEMICAL ABSTRACTS, vol. 89, 1978, abstract no. 117545k, Columbus, Ohio, US; & JP-A-78 31 582 (DAIKIN KOGYO CO., LTD) 24-03-1978**

(73) Proprietor: **AUSIMONT S.p.A.**
**Foro Buonaparte, 31**
**I-20121 Milano(IT)**

(72) Inventor: **Brunetta, Fabio**
**1, via Zanini**
**I-31041 Cornuda Treviso(IT)**
Inventor: **Bader, Stefano**
**44, via Gorizia**
**I-20066 Melzo Milan(IT)**
Inventor: **Pantini, Giovanni**
**74, via Teodosio**
**I-20121 Milan(IT)**

(74) Representative: **Whalley, Kevin et al**
**MARKS & CLERK 57/60 Lincoln's Inn Fields**
**London WC2A 3LS(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services

## Description

This invention relates to perfluoropolyether compositions which are particularly suitable for application in the field of cosmetics and dermatology, which comprise, as the essential component, a perfluoropolyether liquid dispersed in an aqueous or organic phase.

It is well known in the literature to prepare oil/water emulsions in which the oil comprises a perfluorinated compound. These emulsions were chiefly prepared in order to provide synthetic plasma, exploiting the high solubility of oxygen and of carbon dioxide in the perfluorinated compounds which, therefore, act as oxygen carriers. Certain known perfluorinated compounds are perfluorinated cyclo-alkanes (preferably with two or more condensed cycles), heterocyclic perfluorinated compounds, and perfluorinated amines, while compounds having the structure of perfluoropolyethers have proved to be little suited to this kind of application. JP-A-60-034730 discloses perfluoropolyether emulsions comprising a) a perfluoropolyether b) a compound containing perfluoro alkyl groups and functional groups c) a non-ionic surface active agent d) a hydrophilic organic solvent and e) water.

In preparing these emulsions, two main difficulties were met, which consisted in the choice of an emulsifier effective for the perfluorinated compounds, and in the obtainment of sufficiently stable products. As regards the emulsifiers, the best results were obtained with the so-called pluronic polyols (non-ionic emulsifiers with the chemical structure of polyalkyloxanes and prepared starting from mixtures of ethylene oxide and propylene oxide in a suitable ratio); as an alternative, perfluorinated emulsifiers were also used, which, however, were not capable of performing the other functions performed by the pluronic polyols in the synthetic plasma.

The problem of the stability was partially solved by using together different types of perfluorinated compounds (the addition of perfluoroamines is useful to stabilize the emulsions); however, the emulsions obtained so far have to be preserved in cold conditions.

It has now surprisingly been found that it is possible to prepare stable dispersions of perfluoropolyether liquids in water or in an organic liquid immiscible with the perfluoropolyether by using, as a dispersant for the perfluorinated liquid, an emulsion of oil in water (O/W) or of water in oil (W/O), or a solid phase suited to form a gel, suspended in an organic liquid (in the last case, the solid phase is a substance capable of forming a reticular structure by formation of links of the hydrogen-hydrogen type or links by Van der Waal forces). The emulsions and dispersions according to the present invention may be suitably prepared by methods well known in the art.

Accordingly, the present invention provides a composition characterized by comprising a perfluoropolyether liquid dispersed in an oil/water (O/W) emulsion or in a water/oil (W/O) emulsion, or in a solid phase able to form a gel, suspended in a liquid organic phase.

Due to the film-forming characteristics of the perfluoropolyether component, the compositions of the invention have the property of forming a transparent and water-repellent liquid film having, further, permeability to oxygen and other gases, which remains on the surface, to which it has been applied, during relatively long periods of time.

The water-repellency characteristic of the liquid film renders the compositions particularly suitable for applications in the field of water-repellent cosmetics and for high-efficiency applications in the field of the skin-conditioning treatments. As compared with cosmetics of the conventional type, the water-repellent cosmetics offer the great advantage of retaining the cosmetic effect for long times and of making unnecessary frequent retouchings which, conversely, are necessary in the case of the conventional cosmetics.

In skin-rehydration treatments it is highly desirable to have available stable, cosmetically acceptable (non-oil, non-tacky, etc.) compositions which, besides the property of carrying to the subcutaneous layers, and to make available therein water amounts which are sufficient to obtain the desired moistening effect, are also capable of exerting a "barrier" effect towards the outside, without adversely affecting, however, the skin respiration.

In spite of the fact that the skin respiration process exhibits several aspects which are still to be clarified, it is clear, however, that the absorption of oxygen and the elimination of carbon dioxide are of essential importance for the health of the skin.

As already mentioned herein, the compositions according to the invention comprise a perfluoropolyether liquid dispersed in an aqueous or organic phase immiscible with said liquid. More particularly, the perfluoropolyether liquid is maintained dispersed (or emulsified) in a liquid phase through an (aqueous or organic) phase present as an emulsion in the dispersing liquid phase or through a solid phase able to form a gel, suspended in a liquid organic phase.

The O/W emulsion is preferably of the non-ionic or anionic type and the W/O emulsion is preferably of

the non-ionic type.

In a composition in the form of an ointment or paste the perfluoropolyether is preferably dispersed in hydrogenated castor-oil suspended in a liquid triglycerid.

The emulsion in which the perfluoropolyether liquid is dispersed is an oil/water emulsion (O/W) or a water/oil emulsion (W/O) of the conventional type, preparable according to known techniques. In particular, the city phase immiscible with water is suitably selected from among fatty acid esters, hydrogenated and non-hydrogenated vegetable oils, linear hydrocarbons of different length, and ramified hydrocarbons containing for example from 14 to 36 carbon atoms.

The emulsifiers are selected as a function of the oily substance; they may be of the anionic, cationic or non-ionic type.

In the above-mentioned O/W and W/O emulsions, the perfluoropolyether represents a "third phase". Through observations under an optical microscope, which dispersions of perfluoropolyether at 20-30% by weight were subjected to, it was possible to observe the perfluoropolyether phase, which appears in the form of droplets of different sizes, some of which have a larger diameter than those of the emulsified phase (inner phase). The microdrops of the perfluoropolyether compound cannot be coloured, as a result of which they can be distinguished from the drops of the inner phase, which, conversely, can be coloured.

The compositions of the present invention therefore consist of at least three phases immiscible with one another, in which the perfluorinated phase is dispersed in the structure of the emulsified oily or aqueous phase.

The perfluoropolyether dispersions may be prepared according to various methods. The preferred method comprises dispersing the perfluoropolyether in water or in the dispersing organic phase. This step is carried out in the presence of heat (e.g. at 70-80 ° C) and with a mixing turbine running at the maximum speed (e.g. 5,000-10,000 rpm). The inner phase is then added (which may be an aqueous or oily phase), whereafter it is cooled under stirring (in like manner as for the preparation of conventional emulsions).

On the other hand, by adopting the technique of adding the perfluoropolyether to the O/W or W/O emulsions when they are already prepared and cooled down, good dispersion of the perfluoropolyether is not obtained: the droplets have a rather large diameter, higher by 10-20 times than that of the microdrops of the inner phase.

The compositions so prepared have a high stability. Accelerated ageing tests by means of thermoregulation at 40 ° C alternated with cooling in a refrigerator at 4 ° C in alternated cycles of 24 hours each during one week, and allowing then to stand for 4 months, have proved that the perfluoropolyether does not demix, not even partially.

Centrifugation tests (5000 rpm during 30 minutes) have proved that the dispersions in the W/O or O/W emulsions are stable even under these conditions.

The perfluoropolyether for preparing the emulsion or dispersion according to the present invention is preferably present in an amount of from 0.05 to 30 parts for 100 parts of the total sum of the other components. More preferably from 0.2 to 5 parts of perfluoropolyether for 100 parts of the total of the other components are used for preparing the emulsion or dispersion. The parts are by weight.

The perfluoropolyethers utilizable in the compositions of the invention are suitably compounds which contain perfluoroalkylene oxide units or perfluoroxetane rings.

In particular, the repeating units are suitably chosen from the following:

a) $C_2F_40$ and $CF_20$ statistically distributed along the chain;

b) $C_2F_40$, $C_3F_60$ and CFXO (X = radical F or $CF_3$) statistically distributed along the chain

c) $C_3F_60$ and $C_3F_6O$ copolymerized with CFXO (X = radical F or $CF_3$) statistically distributed along the chain;

d) oxetane rings

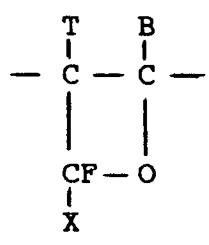

or

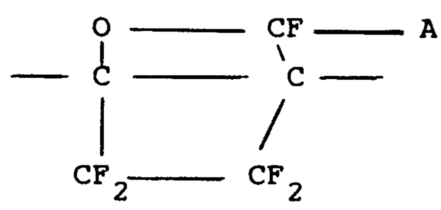

in which B, T and X and A are the same as or different from each other and are perfluoro-oxyalkyl, perfluoropolyoxyalkyl or perfluoroalkyl radicals.

The end groups of the perfluoropolyethers may be like or unlike each other and may be selected in particular from radicals F, $CF_3$, $C_2F_5$, $C_3F_7$, Br, or from polar groups containing one or more electron donor atoms, or from groups containing one or more aromatic rings, optionally containing heteroatoms, capable of giving rise to coordinated bonds or charge-transfer bonds.

The polar groups are suitably bound to the perfluoropolyether chain through divalent groups $CH_20$, $CH_2$-0-$CH_2$, $CF_2$, or $CF_20$.

The mean numeric molecular weight is generally greater than 500, and ranges in particular from 1,000 to 10,000. The viscosity values ($cm^2/s$) (cSt) at 20°C) are generally in the range of from 0.3 to 50 (30 to 5,000).

Particular examples of perfluoropolyethers are:

a) $CF_30-(C_3F_60)_m(CFXO)_n-CF_2Y$

where X and Y are a radical -F or -$CF_3$, m and n are integers, and the m/n ratio is from 5 to 40. These compounds and a method of preparing them are described in British Patent No. 1,104,482.

b) $C_3F_7O(C_3F_60)_m$-Rf

where Rf may be -$C_2F_5$, -$C_3F_7$, or -$CFHCF_3$,

m is an integer higher than 2, preferably from 10 to 100. These compounds and a method of preparing them are described in US Patent No. 3,242,218.

c) $CF_30(C_3F_60)_m(C_2F_40)_n(CFXO)_q-CF_3$

where X = -F or -$CF_3$, m, n and q are integers, the sum $m+n+q$ = 10 to 300, the ratio n/q = 0.5 to 5, and the ratio $m/(q+n)$ = 0.01 to 0.4. These compounds and a method of preparing them are described in U.S Patent No. 3,665,041.

d) $CF_30(C_2F_40)_p(CF_20)_q-CF_3$

where p and q are integers the same as or different from each other, and the p/q ratio is from 0.5 to 1.5. Examples of these compounds and methods of preparing them are described in U.S. Patent Nos. 3,715,378 and 3,665,041. Examples of perfluoropolyethers containing polar end groups are described in U.S Patent No. 4,094,311 and in Italian Patent Application Nos. 21480 A/84 and 21481 A/84.

e) Compounds having an oxetane structure are described in Italian Patent Application No. 19496 A/85.

f) Perfluoropolyethers comprising ($CF_2CF_2CF_20$) units.

g) Perfluoropolyethers comprising ($CF_2CF_20$) units.

The perfluoropolyethers of classes (f) and (g) may be prepared respectively as described in EP-A-148482 and U.S. Patent No. 4,523,039.

In addition to the neutral perfluoropolyethers indicated above there can be also used perfluoropolyethers with functional end groups, such as those described for example in EP-A-165649 and EP-A-165650, U.S. Patent No. 3,810,874, EP-A-148482, EP-A-151877, or in Italian Patent Application No. 22929A/85.

As already mentioned hereinbefore, the compositions of the present invention give rise to considerable film-forming effects: the liquid film obtained is transparent and permeable to gases. A significant proof of the waterproof effect is furnished by applying a cream according to the invention onto the hands and by successively washing the hands. After washing, the water slides away leaving the skin dry and particularly glossy.

Such effect, contrary to what happens with other water-repellent creams, occurs also when washing is carried out with surfactants.

Due to the above-mentioned properties, the compositions of the present invention are particularly suited to applications in the field of cosmetics and of dermatology.

Examples of these applications are:

a) as protective creams and barrier creams (handcreams, ointments (unguents) or pastes to prevent irritations or dermatitis due to contact; water-repellent creams for dermatitis caused by household surfactants or for occupational dermatitis);

b) in paedocosmetics (child cosmetics) as protective creams or pastes for children, for example in the treatment or prevention of the milk crust of the skin or of the scalp;

c) as sun-products, where the waterproofing effect on the skin secures the permanence of the sunlight-filtering active components;

d) as products against wrinkles and for decorative cosmetics, for example in foundation products, eye-shadows, etc. In this case the presence of the fluorinated compound promotes the flowability and therefore facilitates the spreading of the products, thus avoiding or minimizing the unaesthetic cakings (agglomerations) of the product on the skin; in lipsticks and lipglosses, for example, an improvement of both flowability and glossiness is obtained;

e) as creams for massages : since the perfluorinated compound is not absorbed by the skin, it permits also prolonged massages, allowing furthermore the penetration of active substances, if any;

f) in dermatological applications, as a carrier for the absorption of the drugs.

The perfluoropolyether content in the cosmetic emulsions varies as a function of the kind of use, of the number of daily applications and of the application period. Generally it ranges from 0.5-1% for anti-wrinkle creams to be used every day up to 5-10% for high-protective creams. The persistence of the perfluoropolyether on the skin is rather long; removal occurs by washing or by diffusion on clothes or by natural desquamation of the skin.

The invention will be further described with reference to the following illustrative Examples.

Example 1

Preparation of non-ionic O/W emulsions

The preparation was carried out by using a mixing turbine SILVERSTON L2R.

As perfluoropolyethers there were utilized various types of FOMBLIN Y® having formula

$$CF_3-[(O-\underset{\substack{\diagdown \\ CF_3}}{CF}-CF_2)_n-(O-CF)_m]-O-CF_3$$

manufactured by Montefluos S.p.A. : FOMBLIN YO4 (mean molecular weight 1,500 and viscosity 0.35 $cm^2/s$ (35 cSt) at 20 ° C), FOMBLIN Y25 (mean molecular weight 3,000 and viscosity 2,5 $cm^2/s$ (250 cSt) at 20 ° C) and FOMBLIN YR (mean molecular weight 6,000-7,000 and viscosity 10-20 $cm^2/s$ (1,000-2,000 cSt) at 20 ° C).

Except for slight variations in viscosity, no particular differences caused by the utilization of the three different types of FOMBLIN Y were observed in the final product.

The preparation was accomplished by emulsifying the Fomblin in water at 75 ° C (mixing turbine SILVERSTON L2R at 6000 rpm).

Subsequently, while maintaining the mixing turbine running, there was added the oily phase heated to 75 ° C and containing the emulsifying agents; finally, the preparation was completed under continuous and prolonged stirring.

The formula employed for preparing a series of creams was the following:

| | |
|---|---|
| - water contained in the cream | 76 parts |
| - glycerine | 5 parts |
| - fatty acids $C_{12}$-$C_{18}$ polyethoxylated with 8 molecules of ethylene oxide | 8 parts |
| - liquid triglycerid | 4 parts |
| - cetyl and stearyl alcohols | 7 parts |
| - Fomblin Y, various types | 1-3-5 parts. |

The creams had a viscosity from 25-35 $cm^2/s$ (2500 to 3500 cSt) at 5 rpm (revolutions per minute). Although the viscosity was not high, the creams proved to be stable to accelerated ageing tests.

To increase the viscosity, the percentage of fat phase was increased:

| | |
|---|---|
| - water contained in the cream | 70 parts |
| - glycerine | 4 parts |
| - fatty acids $C_{12}$-$C_{18}$ polyethoxylated with 8 molecules of ethylene oxide | 8 parts |
| - liquid triglycerid | 10 parts |
| - cetyl and stearyl alcohols | 8 parts |
| - Fomblin Y, various types | 1-3-5 parts. |

The viscosity of the emulsion was 90-100 $cm^2/s$ (9,000-10,000 cSt) at 20 ° C. By addition of glyceryl monostearate the emulsion viscosity and glossiness was further improved, which also permitted the concentration of the cetyl and stearyl alcohols to be reduced.

On the other hand, by using ethoxylated cetyl and stearyl alcohols it was possible to obtain highly

stable emulsions:

| | |
|---|---|
| - water contained in the emulsion | 76.5 parts |
| - glycerine | 5 parts |
| - cetyl and stearyl alcohols ethoxylated with 12 molecules of ethylene oxide | 3.5 parts |
| - cetyl and stearyl alcohols | 15 parts |
| - Fomblin Y, various types | 1-3-5 parts |

Example 2

Preparation of anionic O/W emulsions

Following the procedure of Example 1, emulsions according to the following formula were prepared:

| | |
|---|---|
| - water contained in the emulsion | 85 parts |
| - self-emulsifying glyceryl mono- and distearate | 10 parts |
| - glyerine | 5 parts |
| - Fomblin Y | 1-3-5 parts. |

The glyceryl mono- and distearate contained small amounts of alkaline stearates, which rendered the emulsifying agent anionic. The emulsions passed all the accelerated ageing tests, including a centrifugal test (30 minutes at 5000 rpm).

The addition of triglycerids to this type of emulsion resulted in a lower glossiness and a lower stability of the preparation.

Example 3

Preparation of W/O emulsions

W/O emulsions generally tend to be less stable than O/W emulsions: however, the presence of Fomblin Y did not further destabilize the system.

For the tests, Arlacel 481 (HLB 4.5) (sorbitan sesquioleate) was utilized as an emulsifier:

| | |
|---|---|
| - Arlacel 481 | 10 parts |
| - liquid paraffin | 38 parts |
| - beeswax | 2 parts |
| - water contained in the emulsion | 45 parts |
| - magnesium sulphate | 0.7 parts |
| - glycerine | 4.3 parts |
| - Fomblin, various types | 1-3-5 parts. |

Example 4

Anhydrous ointments and pastes

An ointment based on triglycerids and hydrogenated castor-oil was utilized.

The hydrogenated castor-oil exhibited a gelifying effect due to the fact that after melting and subsequent cooling, under stirring and in the presence of oils, it formed a solid reticulated structure, which imparted to the system a rheologic behaviour similar to that of the emulsions.

Castor-oil doses below 15% proved unsuitable for preparing stable compositions.

| | |
|---|---|
| - Hydrogenated castor-oil, m.p. 46°C | 15% |
| - liquid triglycerid | 80% |
| - Fomblin Y25 | 5%. |

The emulsion proved to be rather stable to thermostat and to storage tests on shelves, but highly unstable to centrifugation (5000 rpm, 30 minutes).

A higher stability, also to the test in the centrifuge, was exhibited by the following preparation:

| | |
|---|---|
| - hydrogenated castor-oil | 15% |
| - liquid triglycerid | 77% |
| - glyceryl monostearate | 3% |
| - Fomblin Y25 | 5%. |

On the basis of the preceding formulas, pastes useful for example in paedocosmetics (child cosmetics) were prepared:

| | |
|---|---|
| - hydrogenated castor-oil | 10% |
| - paraffin oil | 50% |
| - zinc oxide | 20% |
| - lanolin | 13% |
| - glyceryl monostearate | 2% |
| - Fomblin Y25 | 5%. |

**Claims**

**1.** A composition characterized by comprising a perfluoropolyether liquid dispersed in an oil/water (O/W) emulsion or in a water/oil (W/O) emulsion, or in a solid phase able to form a gel, suspended in a liquid organic phase.

**2.** A composition as claimed in claim 1, characterized in that the O/W emulsion is of the non-ionic or anionic type and in that the W/O emulsion is of the non-ionic type.

**3.** A composition as claimed in claim 1, in the form of an ointment or paste, characterized in that the perfluoropolyether is dispersed in hydrogenated castor-oil suspended in a liquid triglycerid.

**4.** A composition as claimed in any of claims 1 to 3, characterized in that the perfluoropolyether is present in an amount of from 0.05 to 30 parts by weight, preferably 0.2 to 5 parts by weight, for 100 parts by weight of the total sum of the other components.

**5.** A composition as claimed in any of claims 1 to 4, characterized in that the perfluoropolyether liquid includes repeating units $C_2F_40$ and/or $C_3F_60$ or units $C_2F_40$ and $CF_20$ and optionally $C_3F_60$, or units $C_3F_60$ and units CFXO (X = radical F or $CF_3$) statistically distributed along the chain, or includes perfluoro-oxetane units.

**6.** A composition as claimed in claim 5, characterized in that the perfluoropolyether is selected from compounds having formulas:

a) $CF_30-(C_3F_60)_m (CFXO)_n-CF_2Y$

where Y is -F or $-CF_3$, X is the same as Y, m and n are integers, the m/n ratio is from 5 to 40, and units $C_3F_60$ and CFXO are statistically distributed along the chain;

b) $C_3F_70(C_3F_60)_m-Rf$

where Rf = $-C_2F_5$, $-C_3F_7$, or $-CFHCF_3$, and m is an integer higher than 2;

c) $CF_30(C_3F_60)_m(C_2F_40)_n(CFXO)_q-CF_3$

where X = -F or $-CF_3$, m, n and q are integers and their sum is from 10 to 300, the ratio n/q is from 0.5 to 5, and the ratio is m/(q+n) is from 0.01 to 0.4, and units $C_3F_60$, $C_2F_40$ and CFXO are

statistically distributed along the chain;
d) $CF_3O(C_2F_4O)_p(CF_2O)_q$-$CF_3$
where p and q are integers the same as or different from each other, and the p/q ratio is from 0.5 to 1.5, and the units are statistically distributed along the chain;
e) perfluoropolyethers having an oxetane structure.
f) perfluoropolyethers comprising ($CF_2CF_2CF_2O$) units;
g) perfluoropolyethers comprising ($CF_2CF_2O$) units.

**7.** A composition as claimed in claim 5, characterized in that one or both end groups of the perfluoropolyether chain consist of polar groups or aromatic rings.

**8.** A composition as claimed in any of claims 5 to 7, characterized in that the mean numerical molecular weight of the perfluoropolyether is greater than 500, preferably from 1,000 to 10,000.

**9.** A cream, ointment, paste or other formulation for cosmetics and dermatology, characterized by comprising a composition as claimed in any of claims 1 to 8.

**10.** The use in the field of cosmetic and dermatologic products of a composition as defined in any of claims 1 to 8.

**11.** A process for preparing a cosmetic or dermatologic composition, characterized by comprising dispersing a perfluoropolyether liquid in an oil/water (O/W) emulsion or in a water/oil (W/O) emulsion, or in a solid phase able to form a gel, suspended in a liquid organic phase.

## Revendications

**1.** Une composition caractérisée en ce qu'elle comprend un perfluoropolyéther liquide dispersé dans une émulsion huile/eau (O/W) ou dans une émulsion eau/huile (W/O) ou dans une phase solide, apte à former un gel, en suspension dans une phase organique liquide.

**2.** Une composition selon la revendication 1, caractérisée en ce que l'émulsion O/W est du type non-ionique ou anionique et en ce que l'émulsion W/O est du type non-ionique.

**3.** Une composition selon la revendication 1, sous la forme d'un onguent ou d'une pâte, caractérisée en ce que le perfluoropolyéther est dispersé dans de l'huile de ricin hydrogénée en suspension dans un triglycéride liquide.

**4.** Une composition selon l'une quelconque des revendications 1 à 3, caractérisé en ce que le perfluoropolyéther est présent en quantité de 0,05 à 30 parties en poids, de préférence 0,2 à 5 parties en poids pour 100 parties en poids de la somme totale des autres composants.

**5.** Une composition selon l'une quelconque des revendications 1 à 4, caractérisée en ce que le perfluoropolyéther liquide comprend des motifs répétitifs $C_2F_4$ et/ou $C_3F_6O$ ou des motifs $C_2F_4O$ et $CF_2O$ et, éventuellement, $C_3F_6O$, ou des motifs $C_3F_6$ et des motifs CFXO (X = le radical F ou $CF_3$) distribués statistiquement le long de la chaîne, ou comprend des motifs perfluoro-oxétane.

**6.** Une composition selon la revendication 5, caractérisé en ce que le perfluoropolyéther est choisi parmi des dérivés répondant aux formules:
a) $CF_3O$-$(C_3F_6O)_m(CFXO)_n$-$CF_2Y$
dans lesquels Y représente -F ou -$CF_3$, X est identique à Y, m et n sont des nombres entiers, le rapport m/n est compris entre 5 et 40, et les motifs $C_3F_6O$ ET CFXO sont distribués statistiquement le long de la chaîne
b) $C_3F_7O(C_3F_6O)_m$-Rf
dans laquelle Rf = -$C_2F_5$, -$C_3F_7$, ou -$CFHCF_3$, et m est un nombre entier supérieur à 2;
c) $CF_3O(C_3F_6O)_m(C_2F_4O)_n(CFXO)_q$-$CF_3$
dans laquelle X = -F ou -$CF_3$, m, n et q sont des nombres entiers, leur somme est comprise entre 10 et 300, le rapport n/q est compris entre 0,5 à 5, et le rapport m/(q+n) est compris entre 0,01 à 0,4, et les motifs $C_3F_6O$, $C_2F_4O$ et CFXO sont distribués statistiquement le long de la chaîne;

d) $CF_3O(C_2F_4O)_p(CF_2O)_q\text{-}CF_3$
dans laquelle p et q sont des nombres entiers identiques ou différents l'un de l'autre et le rapport p/q est compris entre 0,5 et 1,5, et les motifs sont distribués statistiquement le long de la chaîne;
e) des perfluoropolyéthers présentant une structure d'oxétane;
f) des perfluoropolyéthers comprenant des motifs ($CF_2CF_2CF_2O$);
g) des perfluoropolyéthers comprenant les motifs ($CF_2CF_2O$).

**7.** Une composition selon la revendication 5, caractérisée en ce que l'un des groupes terminaux ou les deux groupes terminaux de la chaîne perfluoropolyéther sont constituée de groupes polaires ou de cycles aromatiques.

**8.** Une composition selon l'une quelconque des revendications 5 à 7, caractérisée en ce que le poids moléculaire numérique moyen du perfluoropolyéther est supérieur à 500, de préférence compris entre 1 000 et 10 000.

**9.** Une crème, un onguent, une pâte ou une autre formulation pour produits cosmétiques et dermatologie, caractérisés en ce qu'ils comprennent une composition selon l'une quelconque des revendications 1 à 8.

**10.** L'utilisation dans le domaine des produits cosmétiques et dermatologiques d'une composition telle que définie dans l'une quelconque des revendications 1 à 8.

**11.** Un procédé de préparation d'une composition cosmétique ou dermatologique, caractérisé en ce qu'il consiste à disperser un perfluoropolyéther liquide dans une émulsion huile/eau (O/W) ou dans une émulsion eau/huile (W/O) ou dans une phase solide apte à former un gel, en suspension dans une phase organique liquide.

**Patentansprüche**

**1.** Zusammensetzung dadurch gekennzeichnet, daß diese einen flüssigen Perfluorpolyether, dispergiert in einer Öl/Wasser-Emulsion (Ö/W) oder in einer Wasser/Öl-Emulsion (W/Ö) oder in einer festen Phase mit der Fähigkeit zur Gel-Bildung, in einer flüssigen organischen Phase suspendiert, umfaßt.

**2.** Zusammensetzung nach Anspruch 1, dadurch gekennzeichnet, daß die Ö/W-Emulsion von nicht-ionischer oder anionischer Art und daß die W/Ö-Emulsion von nicht-ionischer Art ist.

**3.** Zusammensetzung nach Anspruch 1 in Form einer Salbe oder Paste, dadurch gekennzeichnet, daß der Perfluorpolyether in hydriertem Rizinusöl dispergiert ist, das in einem flüssigen Triglycerid suspendiert ist.

**4.** Zusammensetzung nach einem der Ansprüche 1 bis 3, dadurch gekennzeichnet, daß der Perfluorpolyether in einer Menge von 0,05 bis 30 Gewichtsteilen, vorzugsweise 0,2 bis 5 Gewichtsteilen, bezogen auf 100 Gewichtsteile der Gesamtsumme der anderen Bestandteile,vorliegt.

**5.** Zusammensetzung nach einem der Ansprüche 1 bis 4, dadurch gekennzeichnet, daß der flüssige Perfluorpolyether sich wiederholdende $C_2F_4O$- und/oder $C_3F_6O$- oder $C_2F_4O$-und $CF_2O$-Einheiten und gegebenenfalls $C_3F_6O$-Einheiten oder $C_3F_6O$-und CFXO-(X = Radikal F oder $CF_3$) Einheiten umfaßt, die entlang der Kette statistisch verteilt sind, oder Perfluoroxetan-Einheiten umfaßt.

**6.** Zusammensetzung nach Anspruch 5, dadurch gekennzeichnet, daß der Perfluorpolyether ausgewählt wird von Verbindungen der Formeln :
a) $CF_3O\text{-}(C_3F_6O)_m\,(CFXO)_n\text{-}CF_2Y$
worin Y = -F oder $CF_3$ bedeutet, X gleich Y ist, m und n ganze Zahlen sind, das Verhältnis m/n von 5 bis 40 beträgt und die $C_3F_6O$-und CFXO-Einheiten statistisch entlang der Kette verteilt sind;
b) $C_3F_7O(C_3F_6O)_m\text{-}Rf$
worin Rf = $-C_2F_5$, $-C_3F_7$ oder $-CFHCF_3$ bedeutet und m eine ganze Zahl größer als 2 ist;
C) $CF_3O(C_3F_6O)_m(C_2F_4O)_n(CFXO)_q\text{-}CF_3$
worin X = -F oder-$CF_3$ ist, m,n, und q ganze Zahlen sind und deren Summe zwischen 10 bis 300

beträgt, das Verhältnis n/q zwischen 0,5 bis 5 beträgt, und das Verhältnis m/(q+n) von 0,01 bis 0,4 beträgt, und die $C_3F_6O$-, $C_2F_4O$- und CFXO-Einheiten statistisch entlang der Kette verteilt sind;
d) $CF_3O(C_2F_4O)_p(CF_2O)_q$-$CF_3$
worin p und q ganze Zahlen, die gleich oder voneinander verschieden sind, bedeuten, und das Verhältnis p/q zwischen 0,5 bis 1,5 beträgt, und die Einheiten statistisch entlang der Kette verteilt sind;
e) Perfluorpolyether mit einer Oxetan-Struktur;
f) Perfluorpolyether mit ($CF_2CF_2CF_2O$)-Einheiten;
g) Perfluorpolyether mit ($CF_2CF_2O$)-Einheiten;

**7.** Zusammensetzung nach Anspruch 5, dadurch gekennzeichnet, daß eine oder beide Endgruppen der Perfluorpolyether-Kette aus polaren Gruppen oder aromatischen Ringen bestehen

**8.** Zusammensetzung nach einem der Ansprüche 5 bis 7, dadurch gekennzeichnet, daß das durchschnittliche numerische Molekulargewicht des Perfluorpolyethers größer als 500 ist, vorzugsweise zwischen 1.000 und 10.000 beträgt.

**9.** Eine Creme, Salbe, Paste oder andere Formulierung für die Kosmetik oder Dermatologie, dadurch gekennzeichnet, daß diese eine Zusammensetzung nach einem der Ansprüche 1 bis 8 umfaßt.

**10.** Die Verwendung auf dem Gebiet der kosmetischen oder dermatologischen Produkte einer Zusammensetzung nach einem der Ansprüche 1 bis 8.

**11.** Verfahren zur Herstellung einer kosmetischen oder dermatologischen Zusammensetzung, dadurch gekennzeichnet, daß dieses das Dispergieren eines flüssigen Perfluorpolyethers in einer Öl/Wasser (Ö/W)-Emulsion oder in einer Wasser/Öl (W/O)-Emulsion oder in einer festen Phase mit der Fähigkeit zur Gelbildung, suspendiert in einer flüssigen organischen Phase, umfaßt.